# EUROPEAN PATENT APPLICATION

(11) **EP 2 749 653 A1**
(43) Date of publication of application: **02.07.2014**
(21) Application number: 12199781.1
(22) Date of filing: 28.12.2012
(51) Int. Cl.: C12Q 1/68

(54) **Molecular coding for analysis of composition of macromolecules and molecular complexes**

(71) Applicant: Max-Planck-Gesellschaft zur Förderung der Wissenschaften e.V., 80539 München (DE)
(72) Inventor: Borodina, Tatiana, Dr., 14195 Berlin (DE); Soldatov, Aleksey, Dr., 14195 Berlin (DE); Lehrach, Hans, Prof., 14129 Berlin (DE)
(74) Representative: Arth, Hans-Lothar

(57) **Abstract**

The present invention relates to a method for identification of fragments originating from individual macromolecules (MM) or molecular complexes (MC) in a mixture of fragments of different MM or MC using labeling of MM or MC with oligonucleotide markers comprising the following steps:
a) labeling of MM or MC with oligonucleotide markers wherein each particular MM or MC is labeled with identical oligonucleotide markers and preferentially the different MM or MC are labeled with different oligonucleotide markers and wherein the number of identical oligonucleotide markers is sufficient that after subsequent fragmentation or dissociation of fragments of the MM or the MC each fragment is preferentially labeled with at least one of the oligonucleotide marker;
b) fragmentation or dissociation of MM or MC, wherein step a) and b) are optionally done in parallel;
c) mixing labeled fragments of different MM or MC together;
d) analyzing of fragments and determining the nucleotide sequence of the at least one oligonucleotide marker associated with each fragment;
e) identification of fragments originating from individual MM or MC of fragments based on the fact that fragments associated with different oligonucleotide markers were part of different MM or MC before said fragmentation.

## Description

### Background of the invention

To study macromolecules and molecular complexes, researchers often have to fragment them. Afterwards it is necessary to reconstruct the composition of macromolecules (molecular complexes) before fragmentation. In the present invention we suggest to label macromolecules (or molecular complexes) prior to fragmentation so that the components of each macromolecule (or molecular complex) receive identical codes. By further analysis the code would allow to group together fragments, which belonged to the same macromolecules (or molecular complexes) before dissociation.

Molecular complexes can be of any scale: from proteins consisting of multiple subunits and long nucleic acids molecules to content of cells and cell associates. Basing on this invention we present protocols for next generation sequencing (NGS), which allow to determine haplotype, to analyze whole RNA molecules, and to reveal accurate sequences of the repetitive genomic regions.

Many biological methods are not applicable for analysis of large macromolecules (MM) and molecular complexes (MC) as a whole. MM/MC should be fragmented before being analyzed by those methods. For example, proteins should be digested before mass-spectrometry analysis; nucleic acids - fragmented for preparation of sequencing libraries. There exists a problem of reconstruction of the original content and a structure of MM/MC after analysis of fragments.

### Description of the invention

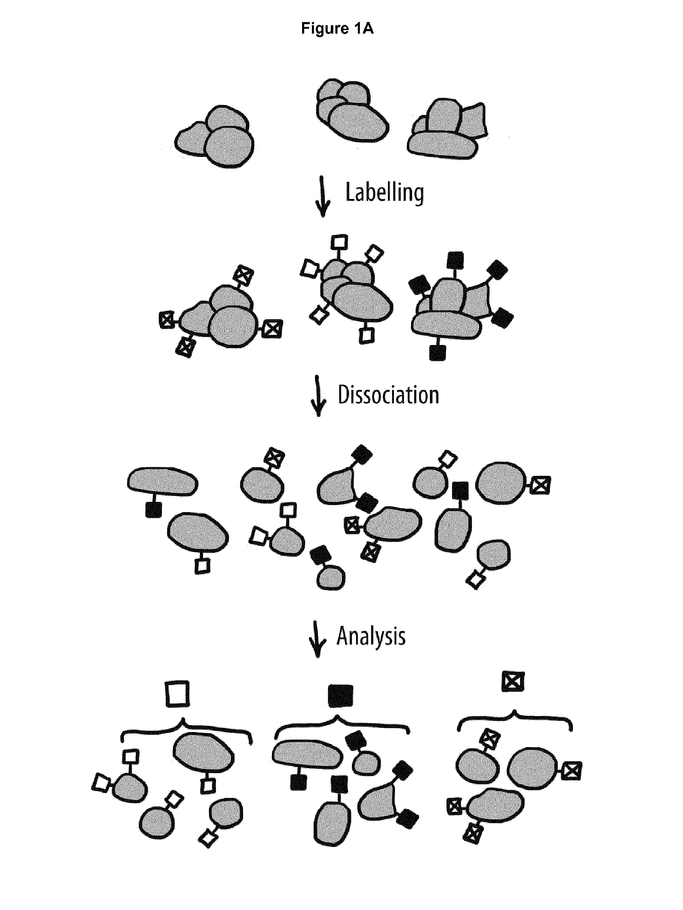

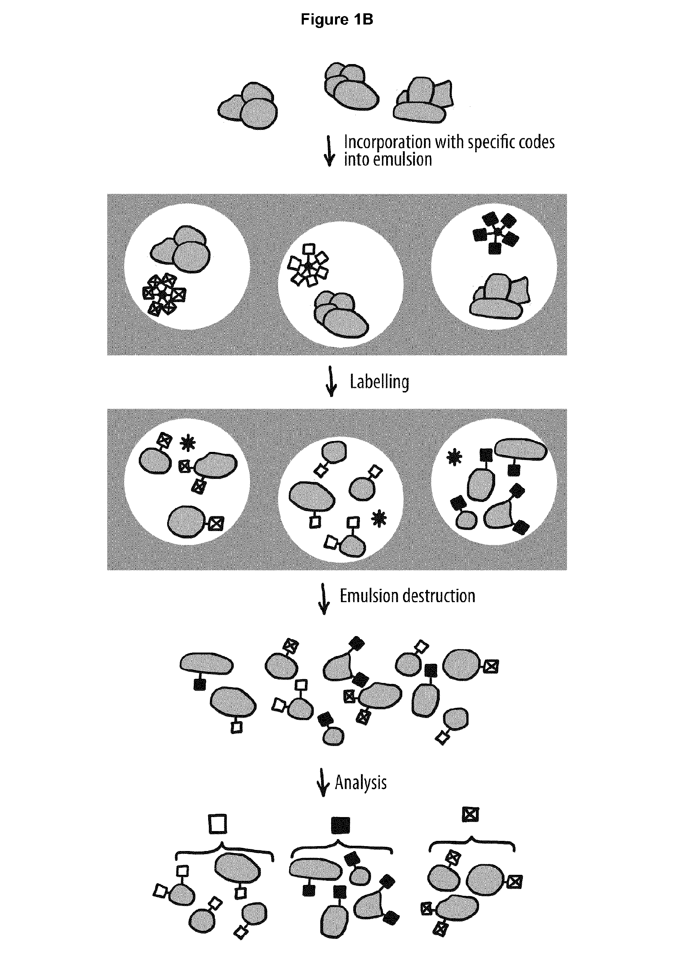

The present invention allows preserving information about the content of MM/MC despite fragmentation and mixing together fragments from different MM/MC. We suggest labeling MM/MC prior to mixing of fragments so that the components of each individual MM/MC receive identical codes. In the subsequent analysis codes allow to group fragments, which belonged to the same MM/MC before dissociation (Figure 1).

For the implementation of the proposed approach it is necessary:
- to have a huge set of code molecules: the number of distinguishable codes should be comparable or larger than a number of individual MM/MC in the analyzed mixture;
- to introduce specifically many of different codes to many of different MM/MC: each individual MM/MC should be labeled by several code molecules with the same code;
- to recognize a single molecule of code on the stage of analysis of MM/MC fragments.

In this invention we suggest several approaches for introduction of specific codes into MM/MC (requirement number 2). The essential part of these approaches is the preservation of MM/MC integrity up to the labeling reaction. We use oligonucleotides with specific nucleotide sequences as code molecules and describe methods for creation of huge set of oligonucleotide codes.

There are several advantages to use oligonucleotides with specific nucleotide sequences as code molecules: (i) individual oligonucleotide molecule may be sequenced (requirement number 3); (ii) comparatively short oligonucleotides are able to provide large variety of nucleic acid sequence variants (codes) , because at each position of an oligonucleotide there can be one of the four nucleotides; (iii) there are a lot of chemical and molecular biology methods for dealing with oligonucleotides (synthesis, cloning, amplification, covalent and non-covalent attachment of oligonucleotides to surfaces and macromolecules) and (iv) it is a common practice to use oligonucleotide sequences as barcodes in large-scale sequencing.

There are special methods of combinatorial chemistry (combinatorial synthesis, synthesis of compounds on microarray) and molecular biology (amplification of library of random molecules) which may be applied for creation of library of oligonucleotide codes (separate sets of oligonucleotide molecules with the identical sequence) on (i) solid supports (microbeads or microarrays) or (ii) directly on MM/MC. This refers to requirement number 1.

We suggest following approaches for introduction of specific codes into MM/MC (requirement number 2):
- spatial isolation of individual labelling reactions in emulsion;
- adsorption on each other the equivalent amounts of code sets and MM/MC (2D adsorption on microarray or 3D adsorption in the diluted solution);
- using MM/MC as carriers for synthesis of a library of codes.

The essential part of all these approaches is keeping the spatial integrity of MM/MC up to the labeling reaction. This provides a possibility for the highly parallel independent labeling of a huge number of MM/MC. The spatial integrity may be preserved either by avoiding fragmentation of MM/MC before labeling or by avoiding dissociation of fragments of MM (fragments/components of MC) before labeling. It is possible to keep fragments of MM (fragments/components of MC) in close proximity with each other in droplets of water-in-oil emulsion, associated with microbeads, associated with each other, etc..

MM/MC may be of the same or different nature as molecules used as codes. That is, the oligonucleotides may be used for coding not only of nucleic acids, but also of protein complexes, nucleic acid-protein complexes and macromolecules of other nature. When the nature of coding molecules and MM/MC is the same, the same approach can be used for determination of the code, and for analysis of the components of MM/MC. If the nature of coding molecules and MM/MC is different, different analysis methods would have to be applied.

Therefore the present invention refers to a method for identification of fragments originating from individual macromolecules (MM) or molecular complexes (MC) in a mixture of fragments of different MM or MC using labeling of MM or MC with oligonucleotide markers comprising the following steps:
a) labeling of MM or MC with oligonucleotide markers wherein each particular MM or MC is labeled with identical oligonucleotide markers and preferentially the different MM or MC are labeled with different oligonucleotide markers and wherein the number of identical oligonucleotide markers is sufficient that after subsequent fragmentation or dissociation of fragments of the MM or the MC each fragment is preferentially labeled with at least one of the oligonucleotide marker;
b) fragmentation or dissociation of MM or MC, wherein step a) and b) are optionally done in parallel;
c) mixing labeled fragments of different MM or MC together;
d) analyzing of fragments and determining the nucleotide sequence of the at least one oligonucleotide marker associated with each fragment;
e) identification of fragments originating from individual MM or MC of fragments based on the fact that fragments associated with different oligonucleotide markers were part of different MM or MC before said fragmentation.

The present invention refers further to a method, wherein labeling of MM or MC with oligonucleotide markers in step a) is performed by mix-and-split combinatorial synthesis of oligonucleotide markers directly on MM or MC. Another preferred embodiment of the present invention is a method, wherein labeling of MM or MC with oligonucleotide markers in step a) is performed by automated parallel synthesis of said oligonucleotide markers directly on MM or MC distributed on a surface. Thereby it is possible that the synthesis of oligonucleotide markers is performed from short oligonucleotides either by ligation or primer extension or from phosphoramidites by chemical synthesis. Another embodiment of the present invention are further methods, wherein labeling of MM or MC with oligonucleotide markers in step a) is performed by attachment of prepared-in-advance oligonucleotide markers to MM or MC by ligation or primer extension or by chemical reactions.

### Application of the invention for NGS sequencing

Fragmented nucleic acids should be used for preparation of NGS libraries, in part because the length of sequencing library molecules is restricted. Besides, sequencing read length is limited. Reconstruction of genomes and transcripomes using those short sequences is a complex task, and obtained results have a restricted value.

Problems appearing during sequencing of genomic DNA:
- *de novo* sequencing: it is difficult to rebuild the full sequence of chromosomes, because it is unclear how to connect unique sequences to repetitive genomic regions;
- resequencing: it is problematic to determine haplotypes (especially, for polyploid genomes) when genomes are reconstructed from short fragments.

These problems make it impossible to determine the exact sequence of chromosomes. Uncertainty is only partly dependent on the accuracy of sequencing itself; the other reason is the ambiguity nature of the assembling of short sequencing reads into the genomic sequence.

For transcriptome analysis it is necessary to determine the composition and the quantity of all transcripts present in the sample. Currently there are difficulties both with structure assessment and gene expression analysis:
- structure: a gene may have several splice variants, alternative promoters and terminators. Reconstruction of a whole transcript using data of short-read sequencing is a complicated task, which currently has no clear solution.
- expression level: it is difficult to accurately estimate the expression level of similar genes on the basis of short-read sequencing. Similarity of genes is a common problem: all genes have two (more in case of polyploid organisms) homologous copies (alleles); repetitive genomic regions produce similar transcripts. Only a portion of reads mapped to the similar genes may be used for comparison of expression levels: namely those reads which overlap sites, different between the homologues. Other reads are useless. This decreases the reliability of expression analysis.

Listed problems lead to "incompleteness" of genome and transcriptome sequencing. It is impossible to be sure that the sequencing experiments would not have to be repeated on another sequencing platform to provide the lacking data.

It is a common opinion that most sequencing problems could be solved by increasing the length of sequenced fragments up to tens of kilobases. The longer the sequencing reads are the easier to assemble them into genome / transcriptome.

In the framework of present invention we suggest to label nucleic acid (NA) molecules before sequencing-related fragmentation and after sequencing to group together sequencing reads originated from individual NA molecules. This allows (on the ranges correspondent to the length of NA molecules before sequencing-related fragmentation):
- to determine haplotypes;
- to link repetitive (homologous for RNA) sequences to the unique ones;
- if the redundancy of sequencing reads originated from particular NA molecule is high enough - to reconstruct not only the content, but also the relative positions of sequencing reads.

Therefore the present invention refers to methods, wherein the MM or MC are nucleic acid macromolecules or complexes which include nucleic acids molecules and wherein step d) comprising sequencing of fragments and oligonucleotide markers associated with said fragments. Furthermore it is preferred that the method according to the invention is applied for genome *de novo* sequencing, resequencing, haplotyping or analysis of transcriptome.

The full sequence of the original NA molecules (before sequencing-related fragmentation) may be reconstructed only at certain conditions: (i) high enough redundancy, (ii) absence of multiple repetitive regions within original macromolecule. But even without reconstruction of relative positions of sequencing reads information about their linkage would significantly facilitate analysis of NGS sequencing data. Information obtained from coded sequencing libraries produced according to the present invention is quite similar to the information produced by first-generation sequencing methods, where long genomic DNA fragments have to be cloned before sequencing. The typical linkage distance reachable by coding of nucleic acid molecules is up to hundreds of kilobases, and may be expanded up to the full-chromosome range for isolates of metaphase chromosomes.

Another aspect is related to the competition of second- and third-generation sequencing platforms. Currently, high-performance second-generation sequencing platforms can produce up to ~200 nucleotides long reads. Despite the price per nucleotide for third-generation platforms is considerably higher, some third-generation platforms have a unique feature: ability to generate longer sequencing reads: up to several thousand or tens of thousands of bases. Present invention allows second-generation sequencing platforms to produce sequencing data linked within the range of hundred thousands of bases and to be competitive with the third-generation machines.

### Haplotyping

One of the main application areas of linkage information is a whole-genome resequencing and haplotyping. Currently resequencing is performed mostly without haplotyping, because existing haplotyping methods are too inconvenient and expensive. Existing haplotyping methods involved:
(1) cloning of long DNA fragments (this method was used for construction of the human reference genome) [9],
(2) isolation of metaphase chromosomes [11],
(3) stochastic separation of fosmid clones or long parental DNA fragments into physically distinct pools [3, 10].

First method produces high-quality data (full-chromosome sequence, excluding highly-repetitive centromere and telomere regions), but is too expensive to be used routinely. Other methods reduce the data output (excluding repetitive regions from the analysis) and simultaneously significantly reduce the price of the analysis.

Using metaphase chromosomes as a starting material it is impossible to reconstruct the sequence of repetitive regions within individual chromosomes.

If parental DNA fragments are separated into physically distinct pools by such a way that "the statistical likelihood of having corresponding fragment from both parental chromosomes in the same pool markedly diminishes" [3], than only sequencing fragments, that uniquely mapped to the reference genome may be successfully haplotyped. Similar to the approach used in the present invention sequencing reads originated from the individual parenteral DNA molecules are grouped together after sequencing. The grouping methods are different. In the present invention grouping is performed on the bases of MM/MC-specific codes only. In the case of [3] grouping is based on two attributes: (i) belonging to the same original physically distinct pool and (ii) the close position of sequencing reads after mapping to the reference genome. Information obtained from coded sequencing libraries produced according to the present invention is quite similar to the information produced when long genomic DNA fragments are cloned before sequencing. In this respect it is quite close to the first method, but with cheap and handy procedure for library production.

### Practical implementations

There are two major approaches in combinatorial chemistry - a technology for synthesizing and characterizing collections of compounds and screening them for useful properties. The term "mix-and-split method" as used herein involves attaching the starting compounds to polymer beads. The beads are then split into groups and reacted with the second set of reagents. After this reaction, all the beads are pooled, mixed together, and split into groups again. The groups of beads are then reacted with the next set of reagents. Additional rounds of pooling and splitting allow libraries with millions of compounds to be generated.

A second method is called "parallel synthesis". All the different chemical structure combinations are prepared separately, in parallel, using thousands of reaction vessels and a robot programmed to add the appropriate reagents to each one. This method is unsuitable for the creation of very diverse libraries but is very useful for the development of smaller and more specialized libraries.

Code in oligonucleotide markers may be (i) a single uninterrupted nucleotide sequence, (ii) a set of nucleotide sequence blocks, subdivided by conservative nucleotide sequence regions; (ii) several nucleotide sequence blocks attached separately to fragments of MM or MC.

Sequencing library molecules have common flanking sequencing library adaptors, which are used for the clonal amplification of the library molecules in the sequencing machine (Illumina, SOLiD).

It is possible to suggest a lot of practical approaches for analysis of MM/MC composition using molecular coding.

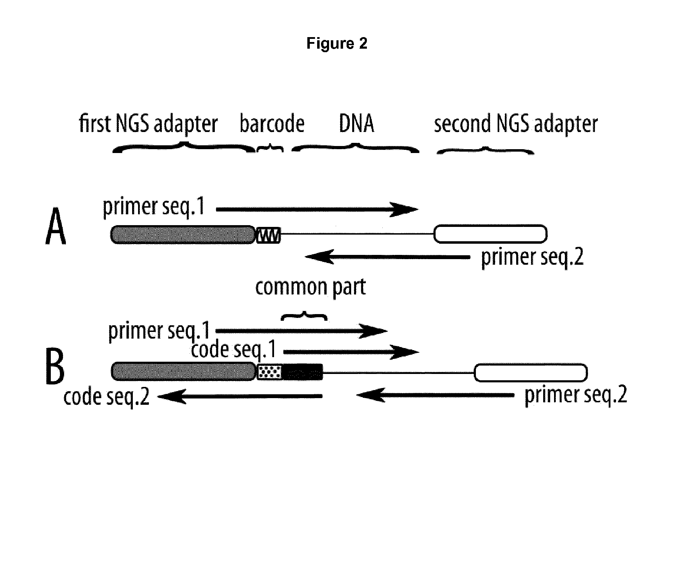

Using of coding oligonucleotides for sorting of sequencing data is well established and can be carried out by standard methods. For example, barcoding is used for the simultaneous sequencing of several libraries. During library preparation a specific oligonucleotide (barcode) is introduced into each molecule. Nucleotide sequences of barcodes are different for different libraries. Barcoded libraries are pooled and sequenced together. Nucleotide sequence of barcode is determined for each fragment (either as an initial part of one of sequencing reads, Figure 2A; or in a separate sequencing reaction using specific sequencing primer, Figure 2B). Nucleotide sequence of barcode allows to assign fragments to particular original libraries.

What is so far non-standard is the introduction of identical oligonucleotide markers in MM/MC. But there are many ways to do it. The proposed approaches are summarised in Table 1. Rows of the table list approaches to create a library of oligonucleotide codes: two methods of combinatorial chemistry («mix-and-split» synthesis and parallel synthesis on a microarray) and one method of molecular biology (clonal amplification, where each single molecule gives rise to an isolated set of identical copies: rolling-circle amplification, bridge-amplification, methods of amplification in emulsion (exponential and linear)). Columns correspond to the methods of association of codes with MM/MC: (i) creation / synthesis of codes directly on the MM/MC and (ii) transfer to the MM/MC of presynthesized codes. For all combinations of "how to create library of codes" - "how to associate codes with MM/MC» it is possible to offer an experimental protocol.

Therefore the present invention refers preferably to methods, wherein oligonucleotide markers are prepared in advance using:
i) mix-and-split combinatorial synthesis from short oligonucleotides by ligation or primer extension or from phosphoramidites by chemical synthesis;
ii) automated parallel synthesis on microarray from short oligonucleotides by ligation or primer extension or from phosphoramidites by chemical synthesis; or
iii) amplification of library of presynthesized oligonucleotides, wherein amplification is based on PCR, RCA, BRSA, bridge amplification.

**Table 1: Methods of coding of MM/MC.**

| | synthesis of codes on MM/MC | transfer of presynthesized codes on MM/MC |
|---|---|---|
| mix-and-split | | |
| microarray | | |
| clonal amplification* | | |

| | | |
|---|---|---|
| * clonal amplification differs from two other methods of synthesis: "mix-and-split synthesis" and "synthesis on microarray" start from certain chemicals, or a limited set of oligonucleotides. For clonal amplification the initial collection of various oligonucleotides (non-amplified library) is required. | | |

### <<mix-and-split synthesis of oligonucleotide codes»-«directly on MM/MC>> (cf. Examples 2-6, 10, 11)

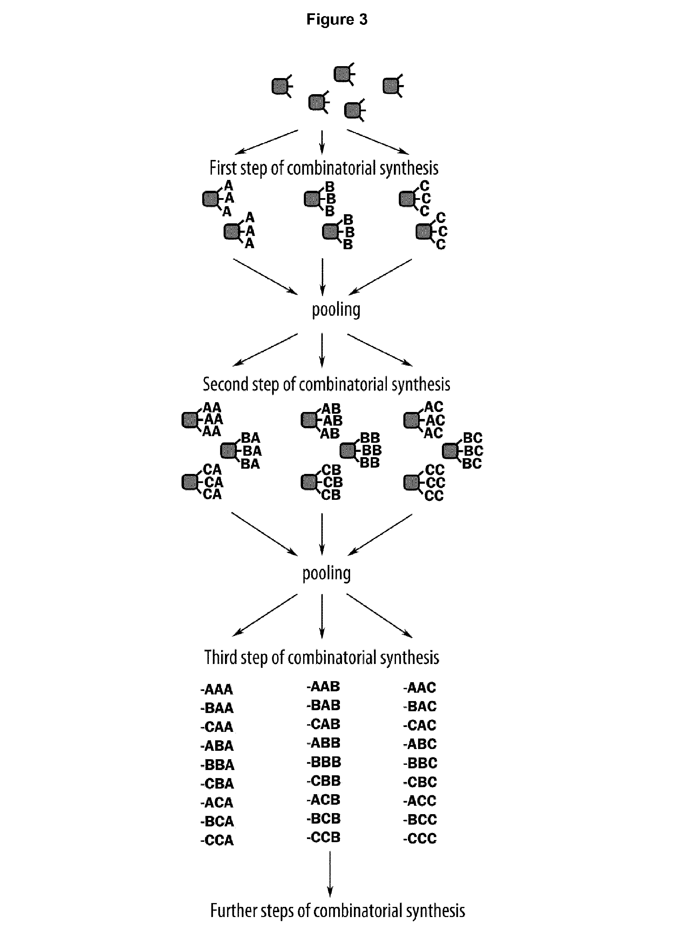

Mix-and-split synthesis is a standard approach of combinatorial chemistry for the synthesis of sets of chemical compounds. The scheme of mix-and-split synthesis is shown in Figure 3. The method works as follows: a sample of support material (carriers) is divided into a number of portions and each of these is individually reacted with a single different reagent. After completion of the reactions, and subsequent washing to remove excess reagents, the individual portions are recombined, the whole is mixed, and may then be again divided into portions.

If to use individual MM/MC as carriers in Figure 3, then on each of them a set of identical codes would be formed. If each of the split stages consists of "n" different reactions, and "k" mix-and-split stages are performed in total, the mix-and-split synthesis would result in n^{k} different codes. If the number of individual MM/MC, participating in the reaction, is much smaller than the number of codes that can be generated, most of the MM/MC would have unique codes, differing from codes on other MM/MC. Then, after the fragmentation of MM/MC, any two fragments bearing the same code are very likely to originate from the same MM/MC.

In combinatorial chemistry chemical synthesis is usually used. For oligonucleotide-based codes, not only chemical but also enzymatic synthesis (ligation or template-directed primer extension) is possible. The advantage of enzymatic synthesis is that it is a "soft" process (if compared to chemical synthesis), which does not damage macromolecules. Chemical synthesis allows only four synthesis variants at each split stage (according to the number of possible nucleotides). For ligation-based code extension, the number of variants (number of parallel reactions at each split stage) can be much larger. If code ligated at each split stage has a length of "n" nucleotides, there are 4ⁿ variants of codes possible. Accordingly the same number (4ⁿ) of ligation reactions may be performed in parallel at each split stage. For "k" stages of ligation-based combinatorial coding 4^{n•k} versions of code can be obtained (Table 2).

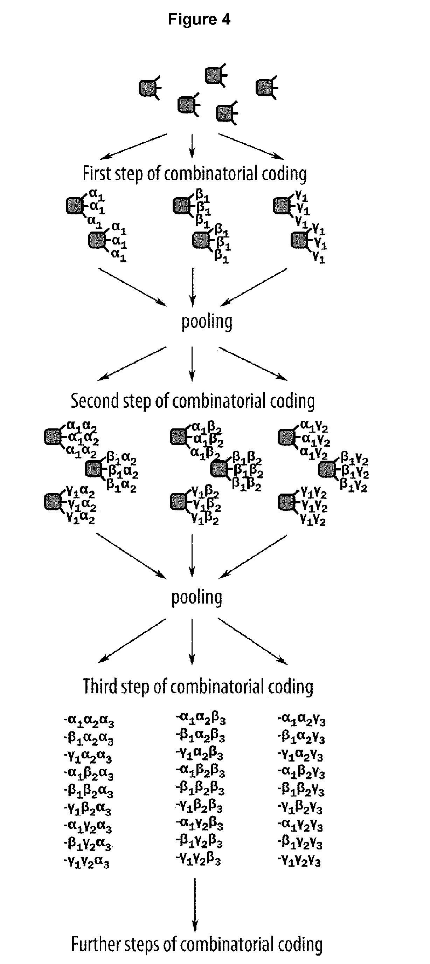

Oligonucleotide adapters may contain not only a code, but also a part that varies from one split stage to another (Figure 4) to reveal incorrectly labeled fragments and exclude them from further analysis. For the "k" stages of ligation-based combinatorial coding 4ⁿ• k different pre-synthesized adapters are required. Table 2 shows the numbers of the resulting codes and required pre-synthesized adapters for specific "n" and "k".

**Table 2: Ligation-based combinatorial coding.**

| Length of coding region | number of codes after'k" cycles of coding | | | | | |
|---|---|---|---|---|---|---|
| | number of different adapters | | | | | |
| | 1 | 2 | 3 | 4 | 5 | 6 |
| 4bp | 256 | 6.6x10⁴ | 1.7x10⁷ | 4.3x10⁹ | 1.1x10¹² | 2.8x10¹⁴ |
| | | 512 | 768 | 1.0x10³ | 1.2x 10³ | 1.5x10³ |
| 5bp | 1024 | 1.0x10⁶ | 1.1x10⁹ | 1.1x10¹² | 1.1x10¹⁵ | 1.2x10¹⁸ |
| | | 2.0x10³ | 3.1x10³ | 4.1x10³ | 5.1x10³ | 6.1x10³ |
| 6bp | 4096 | 1.7x10⁷ | 6.9x10¹⁰ | 2.8x10¹⁴ | 1.2x10¹⁸ | 4.7x10²¹ |
| | | 8.2x10³ | 1.2x 10⁴ | 1.6x10⁴ | 2.0x 10⁴ | 2.5x 10⁴ |

**Table 3: 1µg of ds DNA fragments corresponds to:**

| Length of fragments | number of fragments |
|---|---|
| 100bp | ~10¹³ |
| 1 kb | ~10¹² |
| 10kb | ~10¹¹ |
| 100kb | ~10¹⁰ |
| 1Mb | ~10⁹ |

Ligation-based combinatorial synthesis is capable to provide almost any desired number of codes in a few stages. Table 3 shows the number of fragments of different length in 1 µg of ds DNA. When constructing libraries, it is desirable that the amount of codes is an order of magnitude greater than the number of MM/MC. That is, using adapters with 5-6nt coding regions it is possible in only a few steps (2-5) to obtain the number of codes sufficient for any practical application.

### <<synthesis of oligonucleotide codes on array>>-<<directly on MM/MC>>

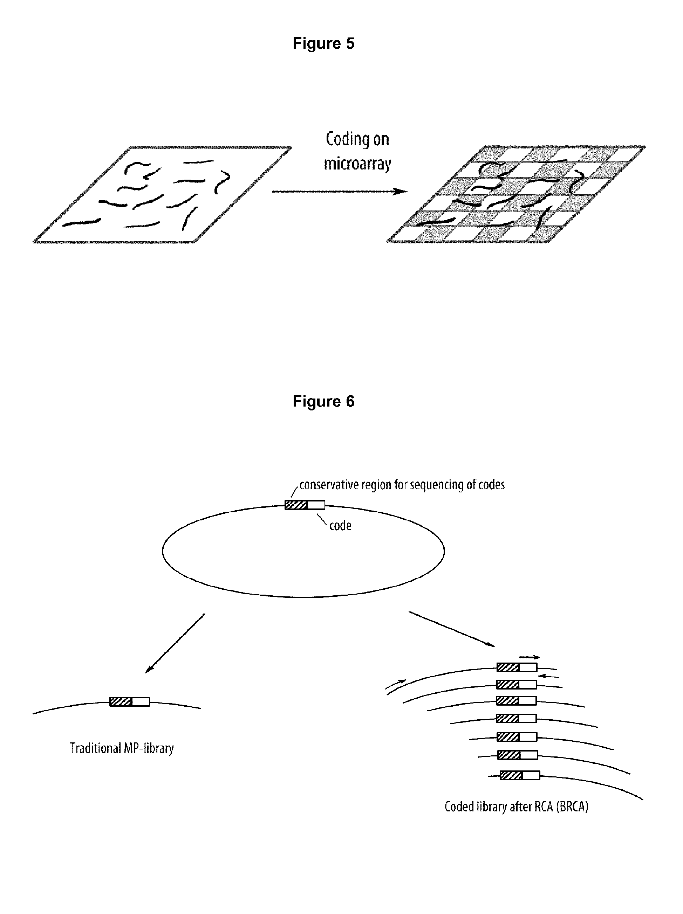

The second standard combinatorial chemistry approach for creating libraries of codes is the synthesis on array. This approach can also be used for the synthesis of codes directly on the MM/MC. If to distribute MM/MC on the 2-dimensional surface so that they rarely overlap with each other and to carry out the synthesis of oligonucleotide codes on such a surface, each component of the particular MM/MC will receive identical codes (or a set of codes that are located close to each other), Figure 5. As in the previous example, the synthesis can be performed either chemically or enzymatically.

### <<clonal amplification>>-<<directly on MM/MC>>

Clonal amplification may be used as alternative method for construction of mate-paired (MP) libraries. Oligonucleotides containing code and conservative region for sequencing of this code are used as adapters for circularisation of the original nucleic acid fragments. Resulting circular molecules are amplified by rolling-circle amplification (RCA), or branched rolling-circle amplification (BRCA). Herewith, both nucleic acid fragments and codes are replicated. Coded concatemers are then randomly fragmented. Only code-containing fragments are selected for construction of NGS-library (for example, by hybridization to an oligonucleotide corresponding to the code-sequencing primer). PE-sequencing and sequencing of codes are performed. Nucleic sequences of codes are used to group clones corresponding to the same original molecules.

MP-library preparation based on clonal amplification has some advantages if compare to traditional protocol. For traditional MP libraries: "original fragment -> 1 library molecule -> 2 sequencing reads". For the described method: "original fragment -> set of library molecules -> multiple reads covering terminal regions of the original fragment", Figure 6.

### Transfer of presynthesised codes on MM/MC

The second column of Table 1 corresponds to experimental approaches, in which the collection of codes is synthesized in advance, and during preparation of coded sequencing library is transferred to MM/MC. Since codes are synthesized in advance, the protocol of library preparation might be shorter and more stable. Collection of codes may be prepared according to the methods listed in rows of the Table 1:
- combinatorial synthesis on microbeads: chemical or enzymatic;
- synthesis on microarray;
- clonal amplification for conversion of single molecules into clones (e.g. bridge amplification on the surface, microbeads in emulsion, etc.)

Some approaches to transfer presynthesised codes to MM/MC are described in the examples 1, 7-9,12,15. In many cases, these approaches are applicable to any way of preparation of collection of codes.

### Technical implementations

One preferred embodiment of the invention refers to methods, wherein oligonucleotide markers are prepared on microarray in a form of spatially isolated groups with identical oligonucleotides and association of particular MM or MC with particular oligonucleotide marker is achieved by adsorption of MM or MC to said microarray.

Further embodiment of the present invention are methods, wherein oligonucleotide markers are prepared in solution as individual oligonucleotide molecules, or as self-associated identical oligonucleotide molecules, or as associates of identical oligonucleotide molecules with microbeads and association of particular MM or MC with particular oligonucleotide marker is achieved in water-in-oil emulsion or by adsorption of MM or MC with said oligonucleotide markers in solution.

Introduction of oligonucleotide markers into MM/MC often involves performing of multiple parallel reactions.

Parallel reactions may be organized in the common reaction solution:
(i) in spatially isolated droplets in in water-in-oil emulsion;
(ii) by adsorption on each other the equivalent amounts of presynthesized oligonucleotyde markers (on microbeads or on microarray) and MM/MC (2D adsorption on microarray or 3D adsorption to beads in the diluted solution);
(iii) by using MM/MC as carriers for synthesis of a library of codes (in combinatorial synthesis, in synthesis on 2D surface (microarray)) or in amplification reaction).

Current robotics and automation also permit to organize a number of phisically separated aliquotes:
- using hydrophilic spots on hydrophobic surface;
- piezo dispensers or other liquid-handling robots;
- RainDance-based approaches;
- etc.

It is inconvenient to add enzymes/chemicals to many separate reactions. It is better to work with a common inactivated mixture and to start reaction after splitting. Reaction may be inactivated by external conditions (for example, decreasing a temperature) or by excluding some key component from the reaction (double valent ions, cofactors, etc.) which is later introduced together with split component (usually, coded oligonucleotides).

For many examples described in this invention large sets of oligonucleotides are required. If oligonucleotides consist of conservative and variable parts and the total number of oligos is too large for the direct synthesis, the collection of oligos might be produced by ligation of common part to locus-specific oligonucleotides. Double-stranded common region may be introduced using ligation-based oligonucleotide synthesis. This is convenient for many applications, because common part is masked from non-specific hybridization.

### Coded libraries

Coded (according to this invention) libraries differ from traditional ones. Traditional libraries consist of completely independent clones, whereas the coded ones - from sets of clones with the same code.

Traditional libraries are prepared preferably with a large excess: number of independent molecules is much larger than the expected number of sequencing reads. Only a small part of the library is sequenced. This helps to minimize the re-sequencing of the same clones.

This approach is not applicable for coded libraries, where the relationship of clones should be revealed. If only a small portion of the library is sequenced, then only a small fraction of existing relationships would be detected. In the extreme case - when just one clone is sequenced from each set of clones with the same code - no relationships between clones would be revealed at all.

The ideal solution would be a complete sequence of the coded library. In practice, it would be necessary:
(i) *non-amplified libraries:* to develop a method of loading of the whole library into a flowcell (without loss of molecules in liquid-handling system and in non-readable regions of a flowcell);
(ii) *amplified libraries:* to find a compromise between the desires (i) to sequence the whole library and (ii) to avoid an unacceptably large number of re-sequencing of the same clones.

The simplest way to compensate for the losses during preparation of the traditional library is to increase the amount of starting material. If the starting material is available in excess then this approach has no negative effects. On the contrary, loss of clones during preparation of coded library is equivalent to the loss of information about components of a MM/MC. Ideal coded library should be constructed from the minimal amount of material with minimal losses.

The critical step, which is sensitive to the demand for "a minimum of material," is the step of fragmentation (dissociation) of MM/MC. Up to this point it is safe to work with excess of material, but before dissociation it is necessary to take as much material as would actually be sequenced, excess is not acceptable. In this respect it is convenient to use for library preparation those methods, which preserve fragment association till the very end of the protocol (whole-genome amplification within water-in-oil emulsion, Exapmle 15; fragmentation without dissociation, Exapmle 10). In this case it is possible
(i) to prepare coded libraries with a large excess as a traditional ones;
(ii) to determine a library titer taking an aliquot of emulsion (bead suspension);
(iii) to take the necessary volume of emulsion (bead suspension) for sequencing.

Coded libraries might be more useful for haplotyping than traditional ones. To reveal that two particular alleles are located on the same chromosome using traditional libraries, they have to be found in the same library molecule. Since only a small part of sequencing reads cover two heterozygous sites at once, only a small part of sequencing data contains information useful for haplotyping. Besides, it is impossible to overcome homozygous regions, which are longer than the fragments used for preparation of PE- (or MP-) libraries. To reveal that two distinct alleles are located on the same chromosome using coded libraries, they have to be discovered in the library molecules with the same code.

This means that:
- if many reads correspond to the same code, it is likely that they would cover many heterozygous sites;
- the length of the parent molecule, corresponding to a particular code may be significantly larger than the length of the fragments used for the preparation of PE-(or MP-) libraries. Therefore, it would be possible to overcome long homozygous regions.

Coded libraries might simplify *de novo* sequencing. Codes permit to reconstruct the content of parental NA molecules. Besides, if coding is associated with NA amplification (Examples 1A, 7) and the redundancy of sequencing reads originated from parental NA molecules is high enough, the relative positions of sequencing reads may be reconstructed - as a result the whole parental NA molecule would be sequenced. In case of presence of multiple repetitive regions within original NA molecule analysis of overlapping parental NA molecules would required for sequence reconstruction.

When using coded libraries for transcriptome research it would be necessary to choose which type of analysis is more important: analysis of the structure or analysis of the expression level, since they have opposite demands to the library construction. To get more detailed information about the structure of transcripts it is desirable that as many library molecules as possible originate from the same RNA molecule - have the same code. However, when analyzing the expression levels, all molecules with the same code should be counted as one original molecule. That is, to increase the statistical reliability of expression analysis it is desirable that as little as possible library molecules have the same code.

It was already mentioned, that it is desirable that the possible number of codes is significantly larger than the number of MM/MC in the sample, since it would reduce the likelihood that independent MM/MC would get the same code. However, useful results can be obtained even when the number of codes is less than or comparable to the number of MM/MC. In this case, some of the MM/MC will get the same codes and extra efforts would be required to understand the linkage of fragments. However, the analysis would still be simpler than it is now, when the sequencing data is analyzed without any additional information about the linkage of fragments to each other.

### Locus-specific sequencing of coded sequencing libraries

It is often required to sequence not the entire genome, but only a certain part of it. Currently the locus-specific sequencing is based on enrichment: oligonucleotides which cover the desired area are synthesized and are used for hybridisation-based selection of relevant clones from the sequencing library. Coded libraries allow another way of locus-specific sequencing: after a low coverage sequencing codes corresponding to the original fragments which overlap area of interest are identified. These identified codes are used for selection of library molecules for further sequencing.

A particular case of locus-specific sequencing - bringing the sequencing project to completeness. Due to the random nature of fragmentation and because of some experimental limitations (like GC-content) it is impossible to obtain an absolutely uniform distribution of sequencing reads. By using codes it is possible to fish out from the library the fragments which correspond to the areas with low coverage.

### Barcoding of combinatorial coded sequencing libraries

In parallel with the coding of individual molecules other parameters of the fragments can be coded too. For example, it is possible to combine coding of molecules with coding of samples (barcoding). Barcodes may be introduced at the earliest stages of the coded library preparation. The samples are then combined and only one library is prepared for the entire project. This approach allows to create one sequencing library for the whole project, to check it with low-coverage sequencing and perform large-scale sequencing only in case of the good library quality.

### Molecular complexes

Another aspect are methods according to the invention applied for analysis of composition of protein molecules and/or protein molecular complexes wherein said complexes which include nucleic acids molecules are aptamers or proximity ligation probes, associated with said protein molecules and/or protein molecular complexes.

Molecular complex is a set of molecules associated with each other. Molecular complexes may have a natural origin (for example, a protein consisting of several subunits) or may be produced during an experiment (for example, a single-stranded nucleic acid molecule with hybridized oligonucleotides).

Depending on the type of the analysis different entities may be understood as a content of the same MM/MC. For example, if peptide-specific aptamers are used for the analysis of multisubunit proteins, then content is "individual protein subunits". If proximity-ligation probes are used for the analysis of multisubunit proteins, then content is "individual protein-protein contacts". In both cases only those "protein subunits" (protein-protein contacts) are analyzed for which user has a specific probe.

Sometimes it is inconvenient to introduce codes directly into intact MM/MC. It might be easier to produce some derivative Molecular Complex (MC), which preserves the association of entities under study, but is more convenient for coding. For example, it is non trivial task to introduce number of codes into double-stranded DNA molecule. In Example 4 this task is solved by conversion of dsDNA into ssDNA with hybridized random primers; in Example 10 this task is solved by conversion of dsDNA into dsDNA fragments attached to microbead.

Molecular complexes can be of almost any scale from proteins consisting of multiple subunits and nucleic acids associates to cells contents and cell associates. For solving of different tasks it might be necessary to analyse the same molecules (for example, genomic DNA), but organized in MM/MC of different nature:
(a) for haplotyping it is possible to use low-fragmented DNA molecules as MM/MC;
(b) for analyzing of spatial distribution of chromosomes within a cell nucleus it is possible to use fragmented nuclear matrix with associated DNA molecules as MM/MC;
(c) for analyzing oncology potential of heterogeneous cancer tumor cells it is possible to use coded cellular DNA as MM/MC.

It is known that cancer tumors are very heterogeneous. Molecular coding allows labeling individual cells. In the subsequent analysis codes would allow to identify components (nucleic acids or proteins), which belonged to the same cell. Thus it will be possible to reconstruct the contents of heterogeneous cells. It would be too expensive to determine the whole genomic sequence of each individual cell, but it is a reasonable task to determine the sequence of all oncogenes within all cells. Currently, to study colocalization of markers cell sorters are used. Colocalization analysis can also be conducted using molecular coding.

Therefore on preferred embodiment are methods of the present invention applied for analysis of composition of individual cells or cell associates wherein said complexes which include nucleic acids molecules are nucleic acids originated from said individual cells or cell associates. It is further preferred that the method according to the present invention is applied for analysis of genotype of individual cells or cell associates wherein complexes which include nucleic acids molecules are DNA molecules originated from said individual cells or cell associates trapped within agarose beads.

Another aspect of the present invention are kits for labeling of MM or MC with oligonucleotide markers according to 1 a) or 1 a-b) or 1 a-c) or 1 a-d).

### Examples

### Example 1A: Preparation of coded NGS library by random primer whole genome PCR amplification.

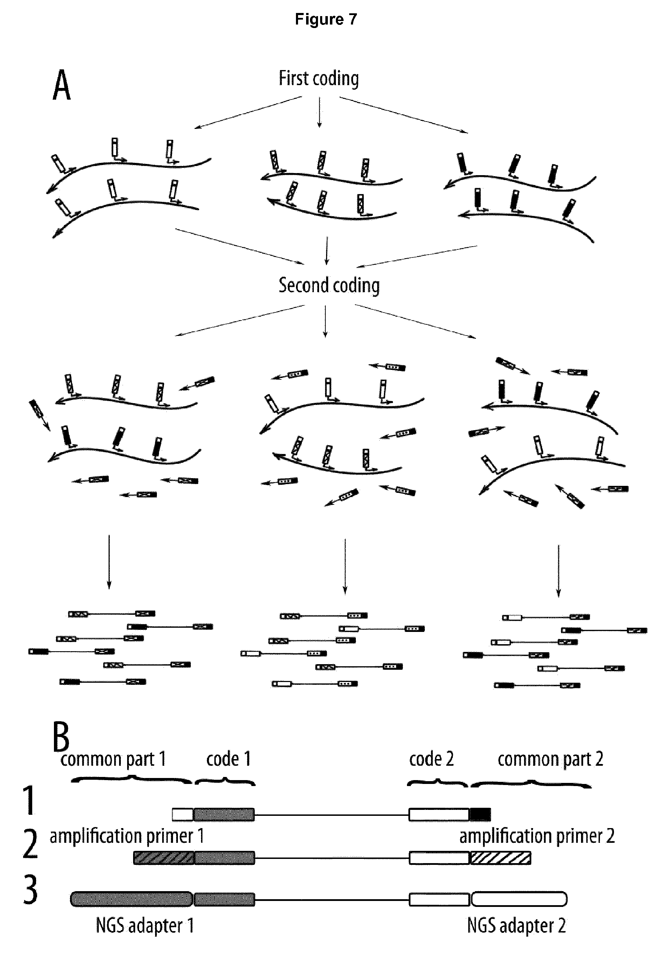

The protocol of preparation of coded NGS library based on a random primer whole genome PCR amplification is shown in Figure 7A. Mix-and-split combinatorial coding is combined with PCR reaction. Coded primers are used in the first two primer extension cycles. It is impossible to use larger number of cycles of combinatorial coding, because, the complex "original molecule - associated primers (annealed or extended)" maintains its integrity only until the second cycle of denaturation. Afterwards, complex "original molecule - associated primers" is denatured and the components of this complex are not associated with each other.

To obtain "N" types of binary combinatorial codes a minimum of a "square root of N" types of primers (and separate split-reactions) for each of two coding steps would be required. That is, if ~10⁶ different binary codes are required (this is a number of 1 Mb ds DNA molecules in 1ng), two oligonucleotide sets each containing ~10³ types of oligonucleotides would have to be used, which is acceptable for the existing methods of oligonucleotides synthesis.

The structure of the molecules obtained as the result of two primer extensions is shown in Figure 7B. If common parts of «first coding primer» and the «second coding primer» are long enough, they can be used for amplification of the library (Figure 7B2) or they can form the complete first and second NGS library adapters (Figure7B3). Besides, the structure shown in Figure 7B2 can be converted into the structure shown in Figure 7B3 by PCR reaction.

### Example 1B: Preparation of coded library by multiplex PCR.

Multiplex PCR is used for the preparation of sequencing library from the definite set of loci. Mix-and-split combinatorial coding may be introduced into PCR reaction as in Example 1A. As a result, it would be possible not only to sequence the selected loci but also to determine the cis / trans location of allelic variants which are separated by distances smaller than the length of template nucleic acid molecules used for PCR reaction.

Large sets of primers may be used in non-coding multiplex PCR: up to thousands of PCR pairs [7]. To perform a two-stage binary coding, each such set should be converted into a collection of sets with different codes. If the total number of primers would be too large for the direct synthesis, the collection of coded primers sets might be obtained by ligation of common coding part to locus-specific oligonucleotides (ligation-based oligonucleotide synthesis). Double-stranded primer region resulting in the ligation-based oligonucleotyde synthesis very nicely blocks common parts of primers preventing non-specific hybridization.

### Example 2: Combinatorial labelling of dsDNA ends.

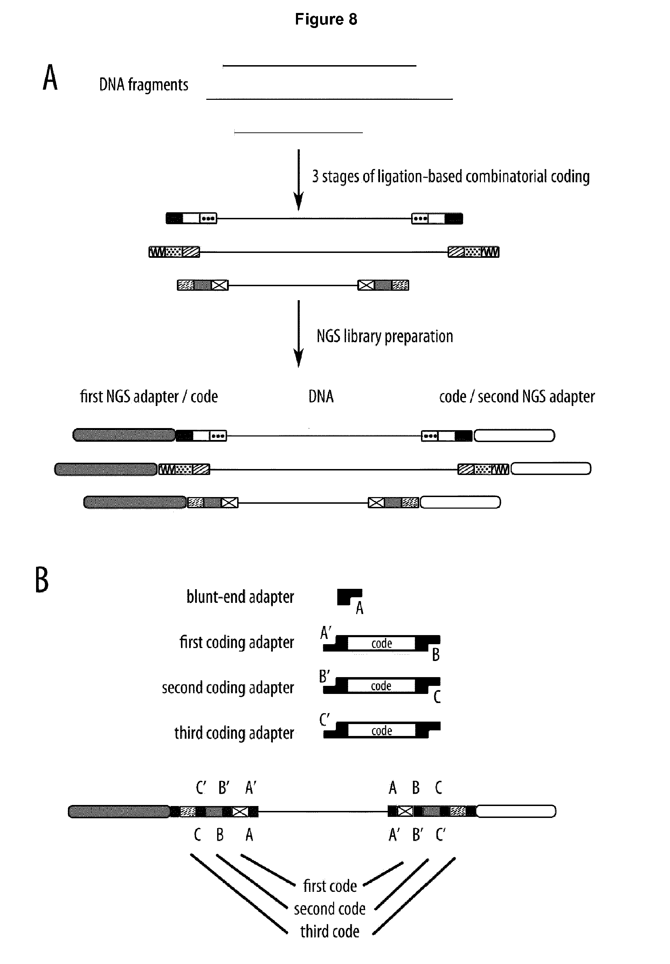

To demonstrate that identical codes are generated on each MM/MC by the mix-and-split combinatorial coding, we have applied the mix-and-split combinatorial ligation for coding of the ends of double-stranded DNA molecules (Figure 8). Afterwards, using the NGS we checked that on both ends of each molecule the same combinatorial codes were formed.

Experimental procedure:
1. shear 1 µg of mouse genomic DNA on Covaris, so that the mean size of fragments is ~400 bp
2. end repair
3. ligate common adapters
4. 3-stage mix-and-split ligation of coding adaptors (CA):
   **1^{st}** stage CA's: a₁, b₁**,** c₁, d₁, e₁, f₁, g₁, h₁, i₁, j₁
   2^{nd} stage CA's: a₂, b₂, c₂, d₂, e₂, f₂, g₂, h₂, i₂, j₂
   3^{rd} stage CA's: a₃, b₃, c₃, d₃, e₃, f₃, g₃, h₃, i₃, j₃
5. preparation of sequencing library
6. PE-sequencing
7. comparison of codes.

The experimental scheme is shown in Figure 8A. DNA is fragmented, ends of the fragments are made blunt and common adapters are ligated to them. Adapters have non-palindromic cohesive ends "A" to prevent ligation of adapters to each other. Ligation of coding adaptors (CA) is performed in three mix-and-split stages. At each stage the mixture is split in 10 separate tubes and in each tube a certain coding adaptor is attached to the ends of DNA fragments. Adapters for PE-sequencing are attached to the coded fragments and the resulting library is sequenced from both ends.

The structure of coding adapters is shown in Figure 8B. To prevent ligation of adapters in the wrong order, adapters for different stages have non-coinciding non-palindromic cohesive ends. Cohesive ends also separate code regions from each other.

The structure of the resulting PE library molecules is shown in Figure 8B. Clones with disturbed structure are excluded from further analysis.

Since different non-palindromic cohesive ends of CA's prevent the ligation of adapters on the wrong stages, then, in principle, it is possible to proceed from one split stage to another without getting rid of non-ligated adapters from the previous stage. Two things should be taken into account:
- ligation of CA's should be as complete as possible;
- there should be a molar excess of CA's on each stage if compare with CA's on the previous stage: CA1 < CA2 < CA3 < CA4 < CA5 < ... If a 1.3-fold molar excess of CA is taken for each stage, the following series of relative amounts of CA would be obtained: 1 < 1.3 1.7 < 2.2 < 2.9 < ...

### Example 3: Preparation of combinatorial coded mate-paired libraries.

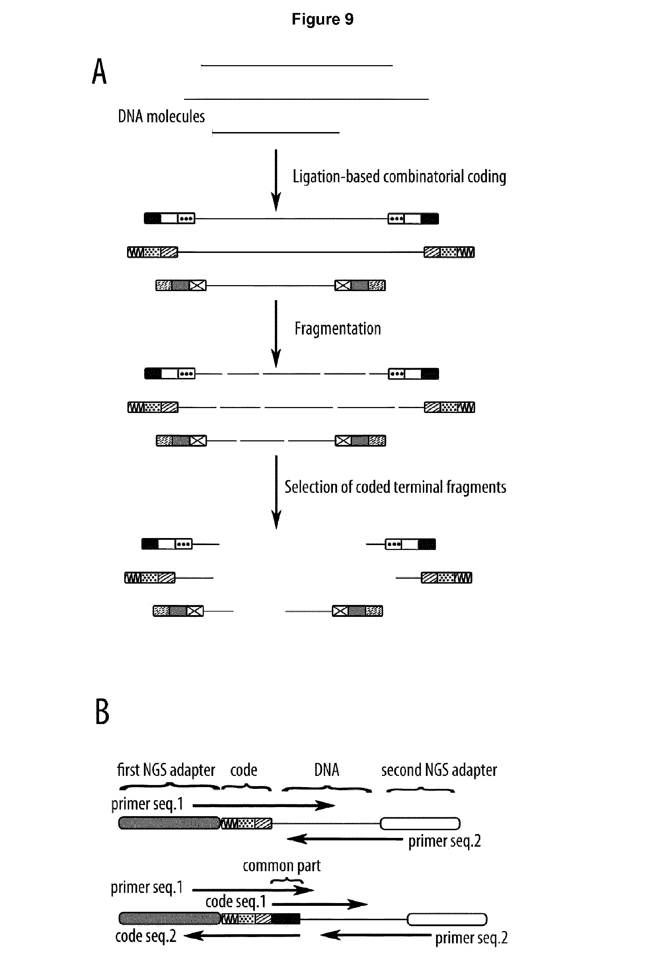

Using the idea of the present invention a new method of MP libraries construction may be suggested. Instead of keeping the ends of DNA molecules physically connected, they can be labelled with the same code. The scheme of preparation of coded MP library shown on Figure 9. After coding (as in example 2), the DNA molecules are fragmented; and only coded terminal fragments are used for construction of sequencing library. By comparing the nucleotyde sequences of codes it is possible to figure out which fragments formed pairs before fragmentation.

The traditional method of construction of MP libraries is inefficient for long initial fragments. Coded MP-libraries may be prepared from any initial fragments which are stable in the solution.

Coded terminal fragments may be selected in different ways:
- using affinity tag included in the code (e.g. biotin);
- by hybridization with oligonucleotides complementary to terminal adapters;
- by nuclease cleavage of fragments without codes (when coding adapters are nuclease-resistant);
- by amplification using primer corresponding to the last ligated adapter.

### Example 4: Preparation of combinatorial coded sequencing libraries.

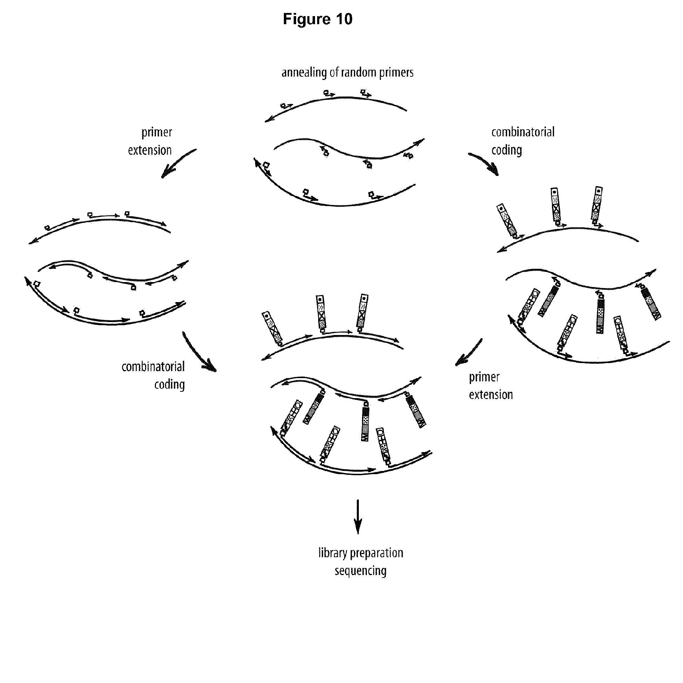

In examples 2 and 3 combinatorial coding is used to label the ends of DNA fragments. A similar approach may be used for labeling the inner parts of the nucleic acid molecules. An example of such a protocol is shown in Figure 10.

On the first step primers with a random 3' part and the predetermined 5' part (designed for attachment of coding adapters) are annealed to the single-stranded nucleic acid molecules.

After «primer extension» and «mix-and-split combinatorial coding» (as in Examples 2 and 3) a molecular complex is obtained, which consists of original nucleic acid molecule and extended random primers, where random primers are marked by identical codes. After dissociation, codes allow to find out which fragments belonged to the same molecular complexes.

Depending on the particular application, it is possible to choose in which order «primer extension» and «mix-and-split coding» operations should be performed.

The approach with extended RP's is applicable both to DNA and RNA molecules (first-strand synthesis by reverse transcriptase).

### Example 5: Coded gap-filling libraries.

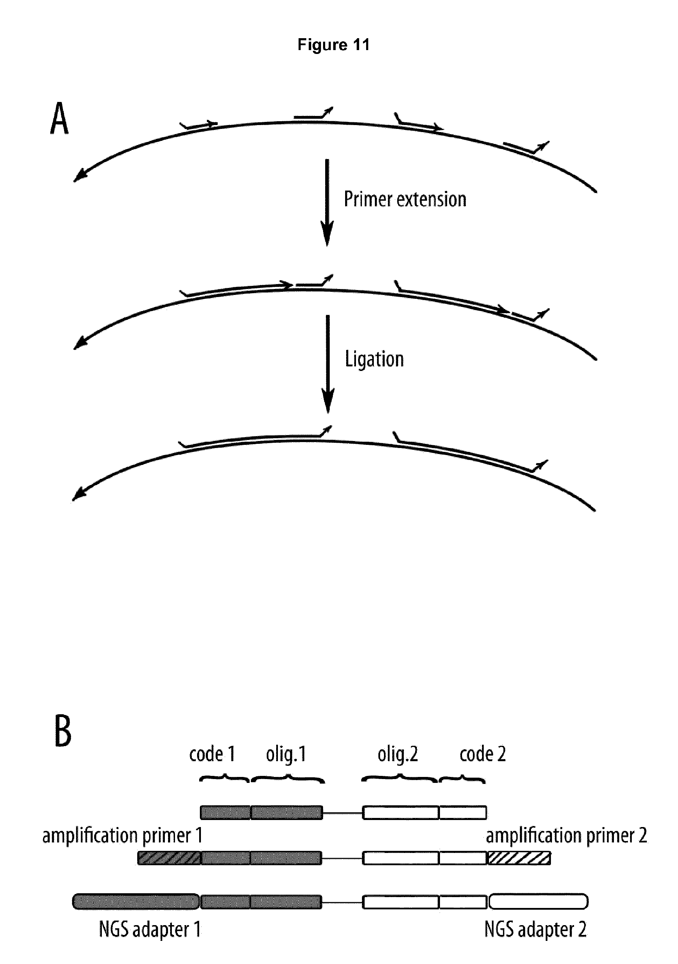

Gap filling - a primer extension followed by ligation - is used, if a specific set of loci needs to be analyzed (a version without primer extension with allele-specific ligation also exists). For each locus two primers are used corresponding to the boundaries of the locus (in contrast to PCR, they are complementary to the same chain), Figure 11. Locus is copied during primer-extension reaction. Elongation product is ligated to the second primer. Using of two specific primers per locus provides high selectivity.

Original molecule and annealed primers remain associated in a complex both during primer extension and ligation reactions. Coding of obtained complexes would make it possible to determine the cis/trans location of allelic variants which are separated by distances smaller than the length of the original nucleic acid molecules (determine haplotype).

Codes may be attached to the primers (to one or both) after hybridization (e.g., using ligation-based combinatorial coding). Besides, binary combinatorial codes, analogous to codes in the Example 1, maybe prepared by using two sets of coded primers. As in the Example 1B set of coded primers can be generated by ligation-based oligonucleotide synthesis. The structure of molecules resulting from the binary coding is shown in Figure 11B.

### Example 6: Combinatorial coded aptamers for analysis of protein complexes.

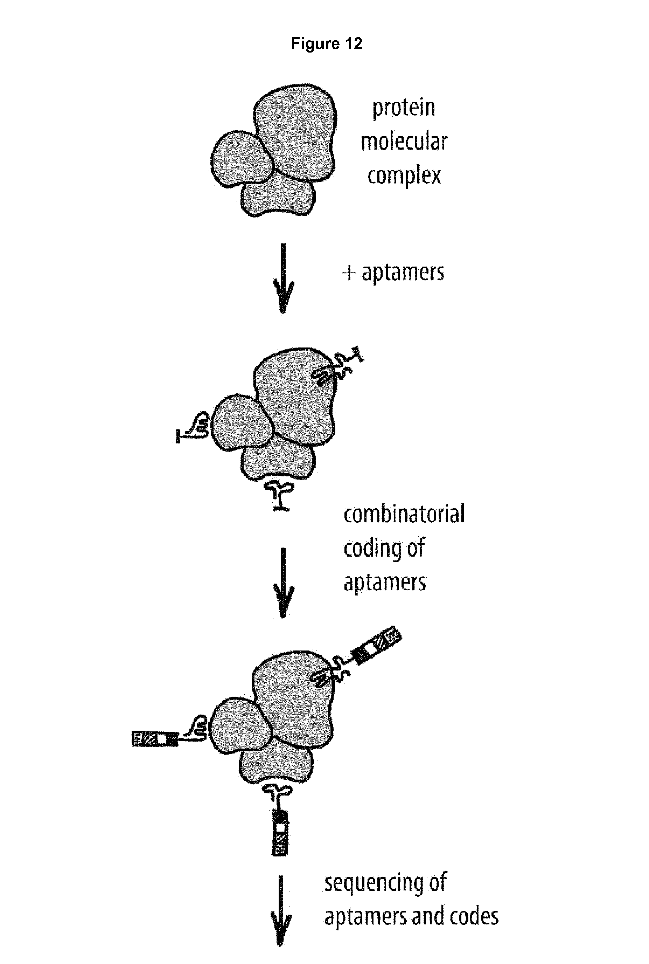

For analysis of protein complexes it is necessary to mark protein subunits. This can be done as shown in Figure 12. Aptamers are attached to proteins, and the resulting complex is labeled using combinatorial approach. After sequencing of codes and aptamers it would be possible to understand which proteins were associated with each other.

### Example 7: Using of coded beads for preparation of coded sequencing libraries (emulsion).

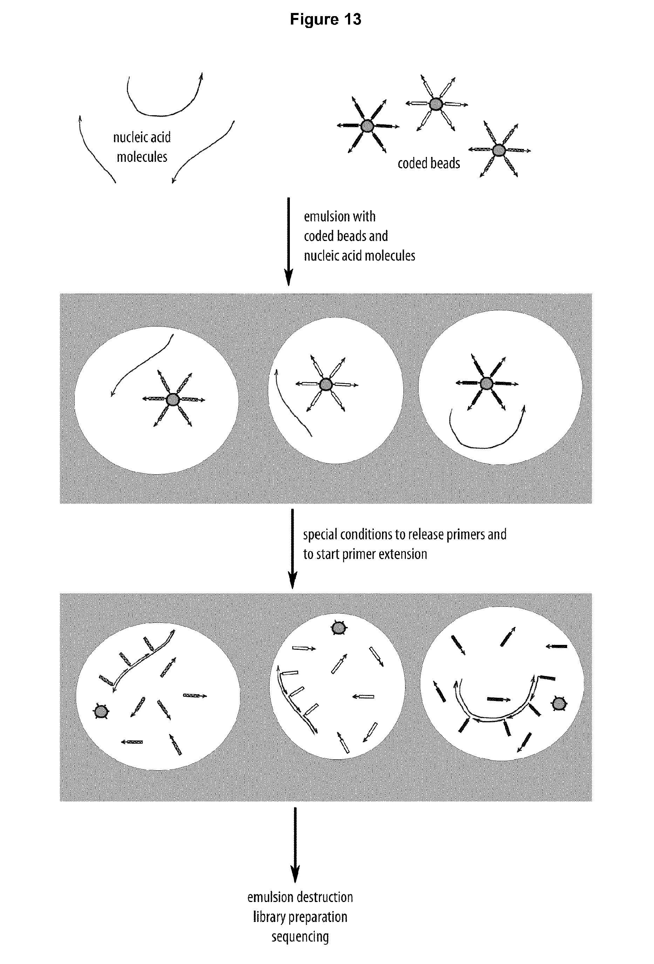

Figure 13 shows a scheme of the preparation of coded library using collection of codes attached to the microbeads. Nucleic acid molecules and microbeads are put into emulsion so that predominantly one bead with a code is associated with one nucleic acid molecule. Then, the external conditions are changed so that the oligonucleotides with codes detach from microbeads, anneal to the nucleic acid molecule and get extended. As a result, in the emulsion droplet a molecular complex is formed, which consists of original nucleic acid molecule and extended random primers, where random primers are marked by identical codes.

### Example 8: Using of coded beads for preparation of coded sequencing libraries (adsorption of nucleic acids on beads).

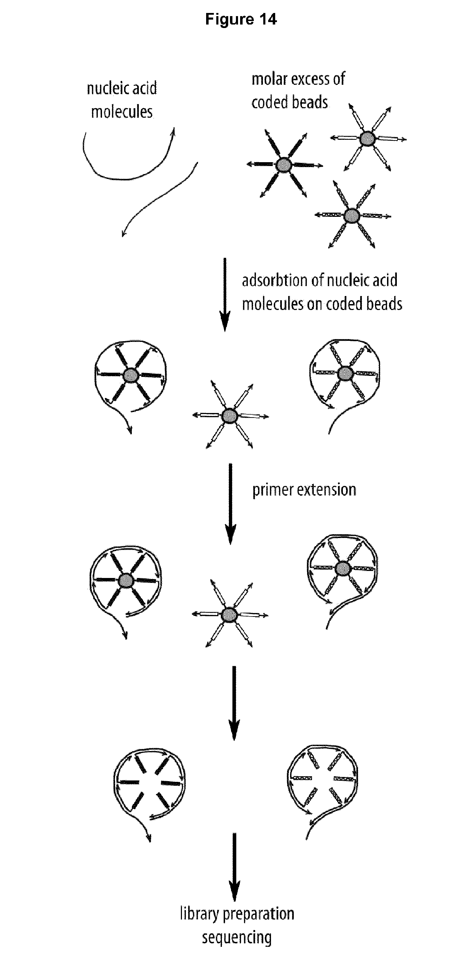

Collection of codes attached to the microbeads can be transferred to the nucleic acid molecules without the use of the emulsion (Figure 14). If adsorption of single-stranded nucleic acids on the microbeads, coated with coded random primers is performed in a very diluted solution, then the individual NA-molecules would be adsorbed on separate microbeads. After the primer-extension reaction on each microbead with the adsorbed molecule a molecular complex would be formed, which consists of original nucleic acid molecule and extended random primers, where random primers would be marked by identical codes.

### Example 9: Proof of principle experiment with two types of coded beads

To demonstrate the possibility to create a coded libraries by adsorbing DNA to the microbeads in diluted solution, the experiment with two types of DNA (from Drosophila and Arabidopsis) and two types of microbeads, covered with coded random primers ("code I" and "code II") was conducted. Each type of DNA was adsorbed to one type of microbeads: Drosophila + "code I" and Arabidopsis + "code II". Then the mixtures were combined with each other and elongation of random primers was performed. Resulting molecular complexes were used for NGS library preparation and obtained clones were sequenced from both ends (PE sequencing). Analysis of the obtained sequences has shown that the Drosophila DNA was always elongated from "code I" primers, and Arabidopsis DNA - from "code II" primers. That demonstrates that in the elongation reaction DNA is associated with only one microbead. If a large collection of coded beads (instead of only two types) is used in the reaction, each DNA molecule would receive a unique code.

### Example 10: Fragmentation without dissociation for preparation of coded libraries.

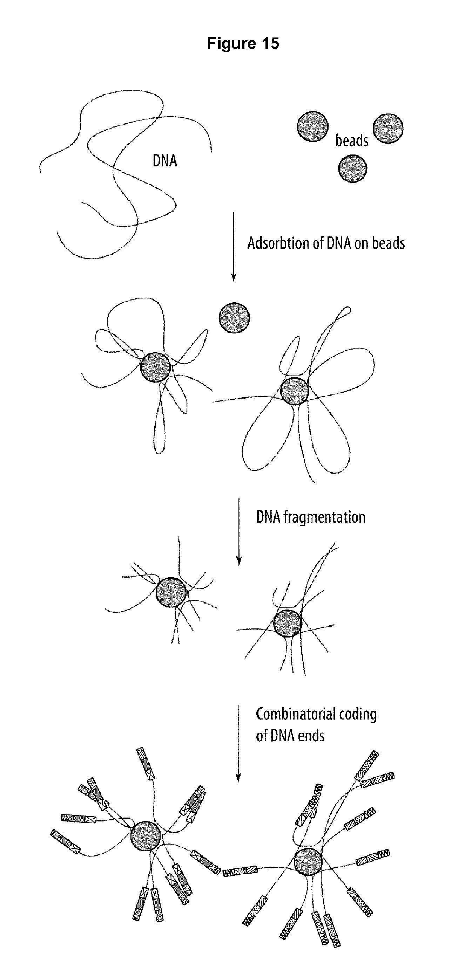

If nucleic acid molecules are adsorbed on some support so that after fragmentation individual parts remain associated with each other, then the coded library can be constructed as shown in Figure 15. If the starting material is double-stranded DNA molecules, after the fragmentation code can be generated at the ends of the molecules by the method described in Example 2.

One of the advantages of this approach - molecules may remain associated with each other until the end of the library construction. Dissociation can be carried out immediately prior to sequencing. This means that, as in the traditional method of NGS-libraries preparation, library can be prepared in excess.

### Example 11: Non direct association of codes with library molecules.

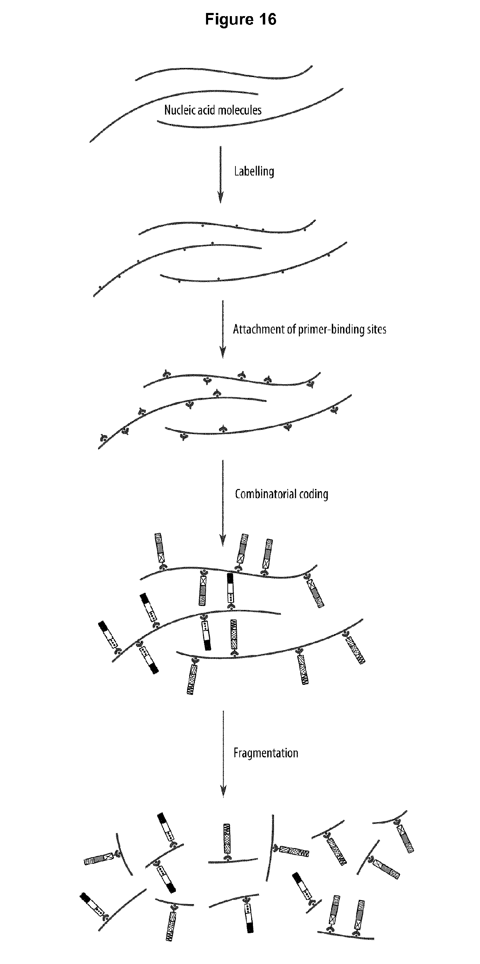

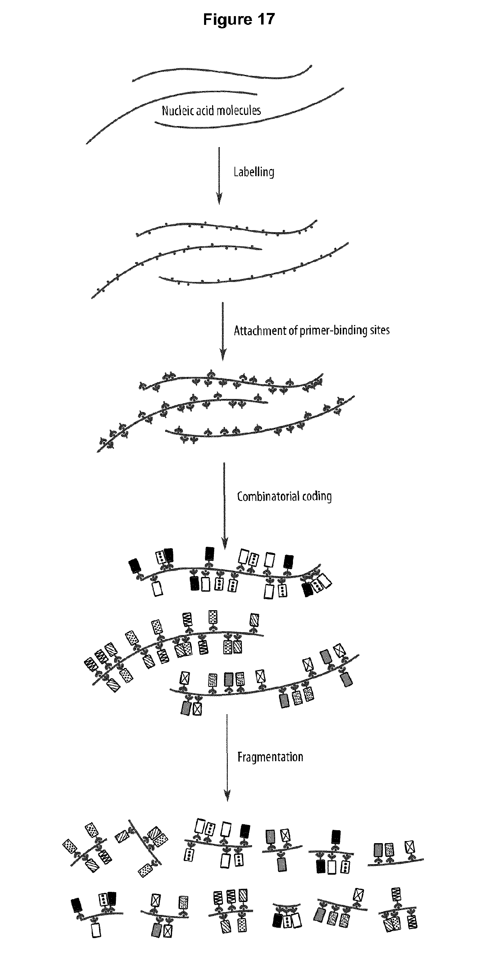

Code does not necessarily has to form a single molecule with MM/MC, it can be only associated with MM/MC. Two examples are shown in Figures 16 and 17. Molecules of biotin are attached to the original nucleic acid molecules. Code associated with streptavidin is attached to biotin molecule. It is possible first to attach a region on which code would be formed, and then generate the code by the combinatorial method as in Example 2, or the presynthesized code may be transferred to the molecule as in Example 7.

For the analysis of such associates a modified NGS platform is required. It should be able to sequence two different molecules at the same position of flowcell: the library molecule itself and the code molecule. Such modifications could be for example:
i. Illumina flowcells, with two sets of primers - for bridge amplification of library molecules and for bridge amplification of codes.
ii. SOLiD beads with two sets of primers: for immobilization of amplified library molecules and for immobilization of codes.

On Figures 16 and 17 codes are generated by combinatorial mix and split method. In Figure 16 during the mix and split synthesis a single molecule of the code is formed. In Figure 17 individual blocks of code (corresponding to different mix and split stages) get associated with the original MM/MC, but do not form a single molecule. In this case, the complete code is a combination of several independent blocks.

### Example 12: Using of microarrays for preparation of coded sequencing libraries.

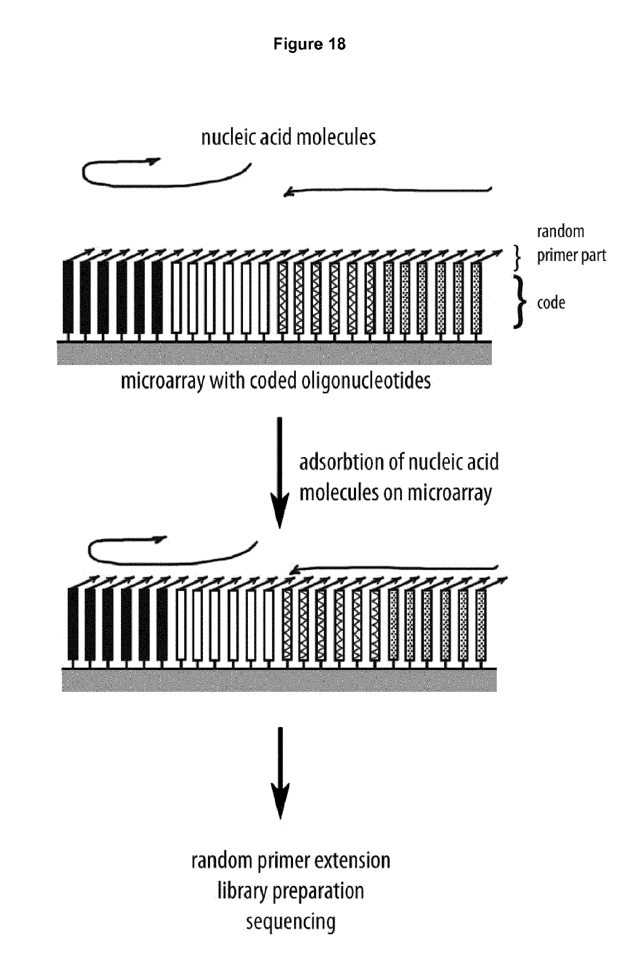

DNA can be adsorbed not only on microbeads (as in Example 8), but also on a microarray (Figure 18), covered with coded random primers. After the primer-extension reaction, each adsorbed nucleic acid molecule would form a molecular complex, consisting of original nucleic acid molecule and extended random primers, where random primers would be marked by identical codes (or by sets of codes located close to each other).

Microarrays have an additional advantage: distribution of codes on the surface is known in advance. This can be used for DNA mapping. If the adsorbed nucleic acid molecule would be stretched along the surface of the microarray, then the codes of extended random primers would change along the molecule in a predictable manner, and would allow to reveal not only fragments belonging to the same initial macromolecule, but also the location of the fragments relative to each other. Given that the 1 kb DNA region has a length of ~0.3 µm, mapping resolution may be in the range of several kb - tens of kb.

### Example 13: Inclusion of NA's into agarose beads.

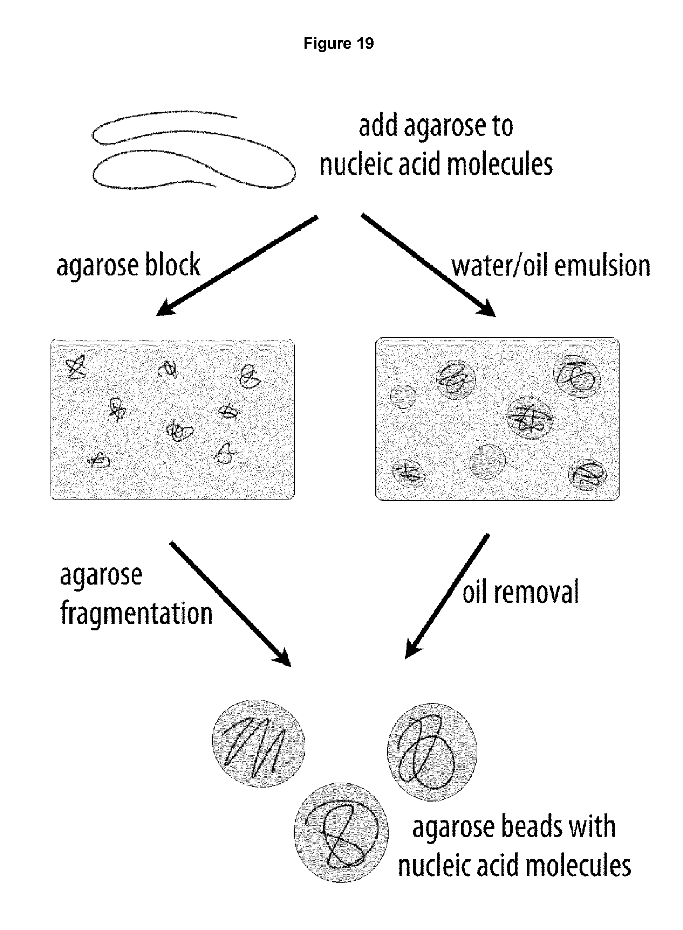

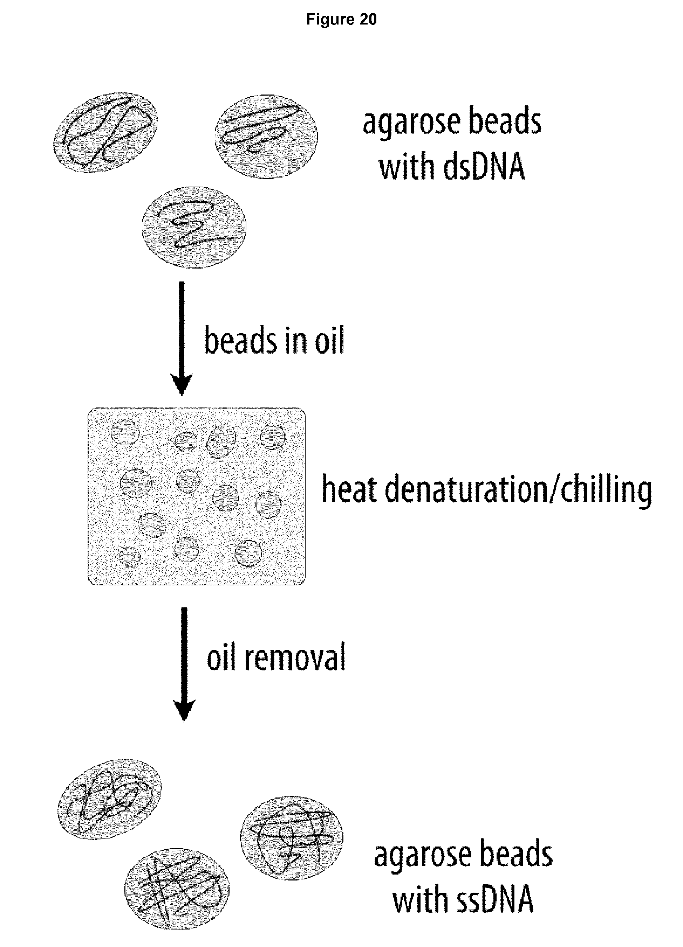

Nucleic acids may be included into agarose beads (Figure 19). As was shown in [8] single stranded nucleic acid molecules are well retained within agarose beads (apparently due to the formation of secondary structure, tangled with agarose fibers). Long double-stranded molecules of nucleic acids should be also well held by the agarose. Beside, double-stranded nucleic acid molecules enclosed in agarose beads, can be converted to single-stranded (Figure 20). Nucleic acid molecules incorporated into agarose beads can be used for molecular coding as described in the previous examples. Agarose beads:
- protect NA molecules from breaking;
- allow to preserve spatial proximity of fragments of slightly sheared molecules;
- offer the advantages of performing reactions on the solid phase: low losses, ease of changing buffers and enzymes.

### Example 14: Inclusion of cellular NA's into agarose beads.

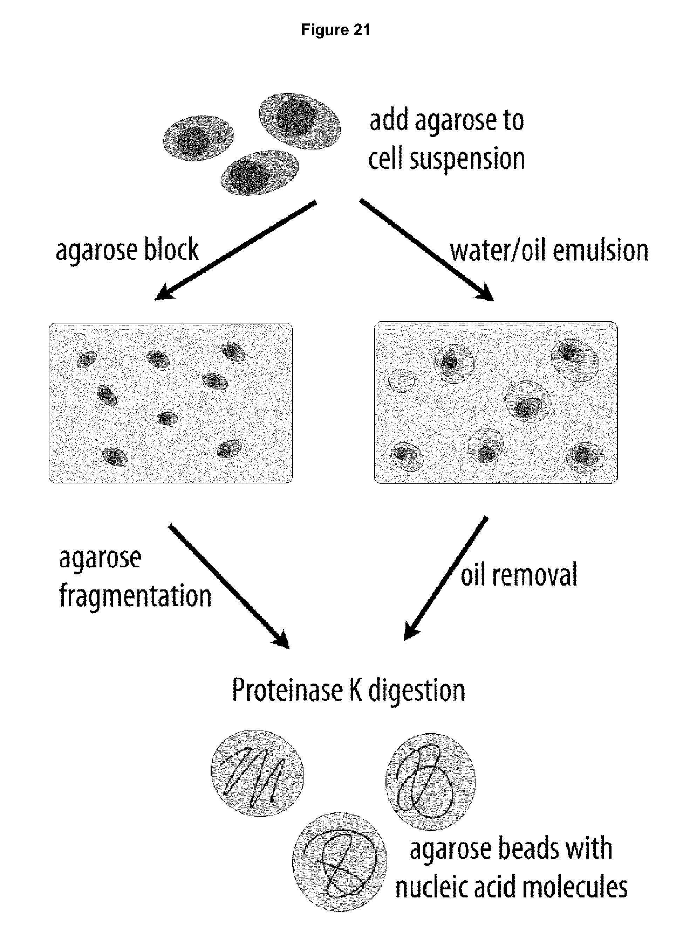

Nucleic acids from individual cells and cell associates may be enclosed in individual agarose beads as shown in Figure 21. Cells in agarose / oil suspension can be lysed by high temperature. After removal of oil and destruction of proteins by proteinases agarose beads containing cellular NA's are obtained. Further manipulations with NA-containing agarose beads are conducted as described in Example 13. Coding of agarose beads containing cellular NA's allows to label NA's of individual cells. In the subsequent analysis codes would allow to identify nucleic acids, which belonged to the same cell.

### Example 15. Preparation of coded NGS library by random primer whole genome PCR amplification in water-in-oil emulsion.

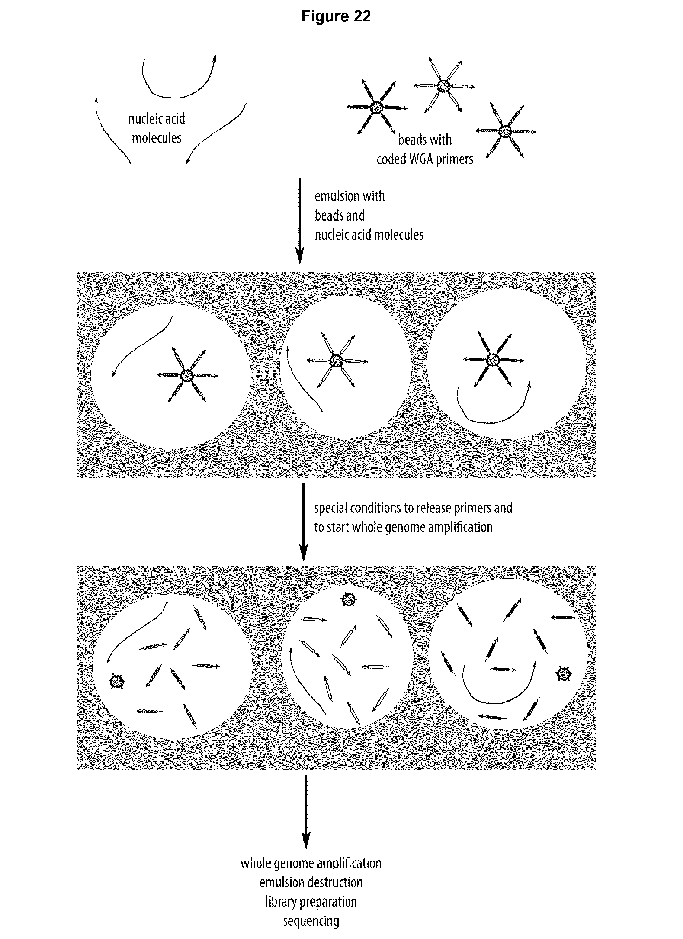

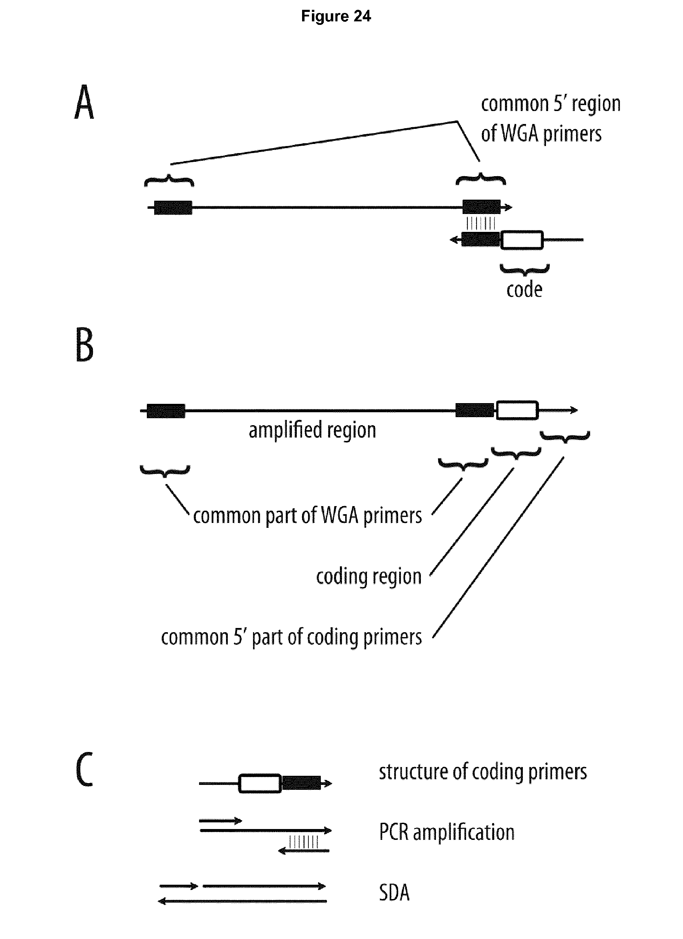

Whole-genome PCR amplification in emulsion permits to isolate spatially amplification of individual parental DNA fragments. Figures 22-24 show schemes of coding associated with amplification in emulsion: 5'-coding on Figures 22 and 23 and 3'-coding on Figure 24.

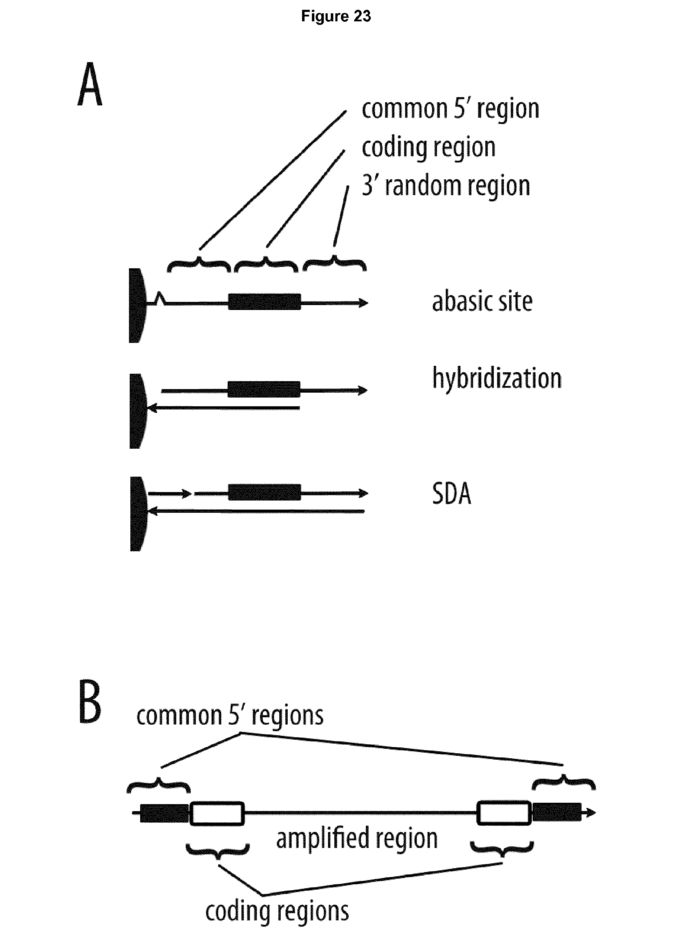

To perform 5'-coding (Figures 22) special coded primers are used for the whole-genome PCR amplification. Coded region is located between conservative 5'-region and random 3' part. Microbeads are used to deliver whole-genome PCR primers with a specific code into individual water droplets. All primers attached to a particular bead have the same code. It is possible to produce such primer-bearing microbeads by mix-and-split ligation-based oligonucleotide synthesis. Microbeads-associated primers are the only source of primers for amplification. Nucleic acid molecules and primer-bearing microbeads are put into emulsion so that predominantly one bead is associated with one nucleic acid molecule. Then, the external conditions are changed so that the oligonucleotides with codes detach from microbeads. Different methods may be used for releasing of primers within water droplets (Figure 23A):
- high temperature: (i) attachment primers to the beads through temperature-sensitive abasic site; (ii) hybridization-based attachment primers to the beads;
- Strand Displacement Amplification (SDA): isothermal, nucleic acid amplification technique based on simultaneous work of nicking endonuclease and strand-displacement polymerase.

The structure of synthesized molecules is shown on Figure 23B. Codes are located between conservative 5'-regions and amplified sequence.

Figures 24 shows how to perform 3'-coding. As a result of whole-genome amplification molecules obtain conservative sequences on both ends. If special primers with codes and with a region complementary to the conservative region of whole genome amplification primers are present within the droplets (Figures 24A), then codes would be attached to the ends of amplified molecules. The structure of synthesized molecules is shown on Figure 24B. Codes are located outside of conservative regions introduced during whole genome amplification.

For 3'-coding whole genome amplification primers are included in water phase of water-in-oil emulsion because they have no codes. Special primers with codes may be delivered into droplets by different ways:
- on primer-bearing microbeads as on Figure 22;
- as single original molecule which should be amplified within the water droplet (by PCR or by Strand Displacement Amplification(SDA)) (Figures 24C).

### References

1. Fosmid-based whole genome haplotyping of a HapMap trio child: evaluation of Single Individual Haplotyping techniques. Duitama J, McEwen GK, Huebsch T, Palczewski S, Schulz S, Verstrepen K, Suk EK, Hoehe MR. Nucleic Acids Res. 2012 Mar;40(5):2041-53. Epub 2011 Nov 18.
2. Whole-genome molecular haplotyping of single cells. Fan HC, Wang J, Potanina A, Quake SR. Nat Biotechnol. 2011 Jan;29(1):51-7. Epub 2010 Dec 19.
3. Accurate whole-genome sequencing and haplotyping from 10 to 20 human cells. Peters BA, Kermani BG, Sparks AB, Alferov O, Hong P, Alexeev A, Jiang Y, Dahl F, Tang YT, Haas J, Robasky K, Zaranek AW, Lee JH, Ball MP, Peterson JE, Perazich H, Yeung G, Liu J, Chen L, Kennemer MI, Pothuraju K, Konvicka K, Tsoupko-Sitnikov M, Pant KP, Ebert JC, Nilsen GB, Baccash J, Halpern AL, Church GM, Drmanac R. Nature. 2012 Jul 11;487(7406):190-5. doi: 10.1038/nature11236.
4. Pacific Biosciences: A new chemistry kit released in 2012 increased the sequencer's read length; an early customer of the chemistry cited mean read lengths of 2.5 to 2.9 kilobases
5. Oxford nanopore: report on sequencing molecules up to 100kb long.
6. Mate Pair Library Preparation protocols for the SOLiD platform:
   - 5500 SOLiD™ Mate-Paired Library Kit, Life Technologies, #4464418
   - SOLiD™ 2x25 bp Mate-Paired Library Construction Kit Life Technologies, #4443472
   - SOLiD™ Long Mate-Paired Library Construction Kit Life Technologies, #4443474 For Illumina platform:
   - Mate Pair Library Preparation Kit v2, Illumina, #PE-112-2002
7. Ion AmpliSeq Comprehensive Cancer Panel, Life Technologies
8. Affinity chromatography of DNA-binding enzymes on single-stranded DNA-agarose columns. Schaller H, Nu"sslein C, Bonhoeffer FJ, Kurz C, Nietzschmann I. Eur J Biochem. 1972 Apr 24;26(4):474-81.
9. The sequence of the human genome. Venter JC, et al. Science. 2001 Feb 16;291 (5507):1304-51. Erratum in: Science 2001 Jun 5;292(5523):1838.
10. Haplotype-resolved genome sequencing of a Gujarati Indian individual. Kitzman JO, Mackenzie AP, Adey A, Hiatt JB, Patwardhan RP, Sudmant PH, Ng SB, Alkan C, Qiu R, Eichler EE, Shendure J. Nat Biotechnol. 2011 Jan;29(1):59-63. Epub 2010 Dec 19. Erratum in: Nat Biotechnol. 2011 May;29(5):459.
11. Long-range polony haplotyping of individual human chromosome molecules. Zhang K, Zhu J, Shendure J, Porreca GJ, Aach JD, Mitra RD, Church GM.Nat Genet. 2006 Mar;38(3):382-7. Epub 2006 Feb 19.

### Description of the Figures

**Figure 1****: A Molecular coding for analysis of composition of macromolecules and molecular complexes:** Labeling is performed in a such way, that each complex obtains identical codes. **B. Molecular coding for analysis of composition of macromolecules and molecular complexes:** Labeling reaction is performed in water-in-oil emulsion. Complexes dissociate during labeling reaction, but water-in-oil emulsion prevents mixing up of codes.
**Figure 2****: Structure of barcoded NGS library molecules:** Arrows correspond to sequencing reads from NGS primers (primer seq.1 and 2) and special primer located nearby with barcode (code seq. 1 and 2).
**Figure 3****: Mix-and-split combinatorial synthesis:** Three steps of combinatorial synthesis are shown, each of them involving the same set of three different reagents.
**Figure 4****: Mix-and-split ligation-based combinatorial coding:** Three steps of combinatorial coding are shown, each of them involving three adapters. Only three different codes: "α", "β" and "γ" are used. Each adapter contains a coding region and step-specific region: "1", "2" and "3". To perform three steps of combinatorial coding nine types of adapters are necessary: "α₁", "β₁","γ₁", "α₂", "β₂","γ₂" and "α₃", "β₃","γ₃". As a result, 27 variants of codes are synthesized.
**Figure 5****: Using of 2D surface for synthesis of codes on MM/MC:** Codes are attached to MM/MC but not to the surface. The surface serves for immobilization of MM/MC (left and right) and as a framework for ordered reagents distribution (right).
**Figure 6****: Clonal amplification for construction of MP-libraries:** Arrows correspond to sequencing reads from NGS primers and a special primer located nearby with a code.
**Figure 7****: Preparation of coded NGS library by random primer whole genome PCR amplification:** A. Two stages of .mix-and-split combinatorial coding. Common 5' ends of the coded primers are shown as white (first primer extension) and black (second primer extension) boxes. B. Structure of molecules after two primer extensions. Common parts may be used for amplification, sequencing, ligation, etc. of the whole molecule pool.
**Figure 8****: Combinatorial labelling of dsDNA ends.** A. Preparation of PE NGS library from fragments with combinatorial codes on both ends. B. Structure (i) of coding adapters used at different stages of ligation-based mix and split coding and (ii) of the final PE library molecule.
**Figure 9****: Preparation of combinatorial coded mate-paired libraries.** A. Scheme of preparation of coded MP library. B. Structure of the coded MP library molecules. Arrows correspond to sequencing reads from NGS primers and a special primer located nearby with a code.
**Figure 10****. Preparation of combinatorial coded sequencing libraries.**
**Figure 11****: Coded gap-filling libraries**. A. Original molecule and extended/ligated primers form a stable complex. B. Structure of binary coded gap-filling library molecules.
**Figure 12****: Combinatorial coded aptamers for analysis of protein complexes.**
**Figure 13****. Using of coded beads for preparation of coded sequencing libraries (emulsion).**
**Figure 14****: Using of coded beads for preparation of coded sequencing libraries (adsorption of nucleic acids on beads).**
**Figure 15****: Fragmentation without dissociation for preparation of coded libraries.**
**Figure 16****: Non direct assotiation of codes with library molecules: Code in single** molecule.
**Figure 17****: Non direct assotiation of codes with library molecules: Distributed** codes.
**Figure 18****. Using of microarrays for preparation of coded sequencing libraries.**
**Figure 19****: Inclusion of NA molecules into agarose beads:** Two variants of NA's inclusion into agarose: (i) fragmentation of agarose gel with included NA's; (ii) preparation of water/oil emulsion with NA's solubilised in hot melted agarose; chilling the emulsion; and washing off the oil from beads.
**Figure 20** **Denaturation of ds NA molecules within agarose beads:** Agarose beads containing double-starnded NA molecules may be placed into emulsion to prevent transfer of NA molecules between beads. During heating of agarose/oil suspension two processes occur simultaneously: (i) denaturation of NA's; (ii) agarose melting. After chilling the emulsion single-stranded NA's get fixed in beads. Besides agarose gel prevent renaturation of NA's.
**Figure 21** **Inclusion of cellular NA's into agarose beads.** Two variants of cells inclusion into agarose: (i) fragmentation of agarose gel with included cells; (ii) preparation of water/oil emulsion with cell suspension in melted low-melting-point agarose; chilling the emulsion; and washing out of gel beads from oil.
**Figure 22** **Preparation of coded NGS library by random primer whole genome PCR amplification in water-in-oil emulsion, 5' coding:** Scheme of the method.
**Figure 23** **Preparation of coded NGS library by random primer whole genome PCR amplification in water-in-oil emulsion, 5' coding:** A. Different methods for releasing of primers within water droplets. B. The structure of synthesized molecules.
**Figure 24** **Preparation of coded NGS library by random primer whole genome PCR amplification in water-in-oil emulsion, 3' coding:** A. Structure of WGA molecules before extension on coding primer. B. Structure of WGA molecules before extension on coding primer. C. Different methods for amplification of primers with codes within water droplets.

## Claims

1. Method for identification of fragments originating from individual macromolecules (MM) or molecular complexes (MC) in a mixture of fragments of different MM or MC using labeling of MM or MC with oligonucleotide markers comprising the following steps:
a) labeling of MM or MC with oligonucleotide markers wherein each particular MM or MC is labeled with identical oligonucleotide markers and preferentially the different MM or MC are labeled with different oligonucleotide markers and wherein the number of identical oligonucleotide markers is sufficient that after subsequent fragmentation or dissociation of fragments of the MM or the MC each fragment is preferentially labeled with at least one of the oligonucleotide marker;
b) fragmentation or dissociation of MM or MC, wherein step a) and b) are optionally done in parallel;
c) mixing labeled fragments of different MM or MC together;
d) analyzing of fragments and determining the nucleotide sequence of the at least one oligonucleotide marker associated with each fragment;
e) identification of fragments originating from individual MM or MC of fragments based on the fact that fragments associated with different oligonucleotide markers were part of different MM or MC before said fragmentation.

2. The method according to claim 1 wherein labeling of MM or MC with oligonucleotide markers in step a) is performed by mix-and-split combinatorial synthesis of oligonucleotide markers directly on MM or MC.

3. The method according to claim 1 wherein labeling of MM or MC with oligonucleotide markers in step a) is performed by automated parallel synthesis of said oligonucleotide markers directly on MM or MC distributed on a surface.

4. The method according to claim 2 or 3 wherein synthesis of oligonucleotide markers is performed from short oligonucleotides either by ligation or primer extension or from phosphoramidites by chemical synthesis.

5. The method according to claim 1 wherein labeling of MM or MC with oligonucleotide markers in step a) is performed by attachment of prepared-in-advance oligonucleotide markers to MM or MC by ligation or primer extension or by chemical reactions.

6. The method according to claim 5 wherein oligonucleotide markers are prepared in advance using:
i) mix-and-split combinatorial synthesis from short oligonucleotides by ligation or primer extension or from phosphoramidites by chemical synthesis;
ii) automated parallel synthesis on microarray from short oligonucleotides by ligation or primer extension or from phosphoramidites by chemical synthesis; or
iii) amplification of library of presynthesized oligonucleotides, wherein amplification is based on PCR, RCA, BRSA, bridge amplification.

7. The method according to claim 5 or 6, wherein oligonucleotide markers are prepared on microarray in a form of spatially isolated groups with identical oligonucleotides and association of particular MM or MC with particular oligonucleotide marker is achieved by adsorption of MM or MC to said microarray.

8. The method according to claim 5 or 6, wherein oligonucleotide markers are prepared in solution as individual oligonucleotide molecules, or as self-associated identical oligonucleotide molecules, or as associates of identical oligonucleotide molecules with microbeads and association of particular MM or MC with particular oligonucleotide marker is achieved in water-in-oil emulsion or by adsorption of MM or MC with said oligonucleotide markers in solution.

9. The method according to any one of claims 1 - 8, wherein the MM or MC are nucleic acid macromolecules or complexes which include nucleic acids molecules and wherein step d) comprising sequencing of fragments and oligonucleotide markers associated with said fragments.

10. The method according to claim 9 applied for genome *de novo* sequencing, resequencing, haplotyping or analysis of transcriptome.

11. The method according to claim 9 applied for analysis of composition of protein molecules and/or protein molecular complexes wherein said complexes which include nucleic acids molecules are aptamers or proximity ligation probes, associated with said protein molecules and/or protein molecular complexes.

12. The method according to any one of claims 9 - 11 applied for analysis of composition of individual cells or cell associates wherein said complexes which include nucleic acids molecules are nucleic acids originated from said individual cells or cell associates.

13. The method according to claim 12 applied for analysis of genotype of individual cells or cell associates wherein complexes which include nucleic acids molecules are DNA molecules originated from said individual cells or cell associates trapped within agarose beads.

14. Kit for labeling of MM or MC with oligonucleotide markers according to step a), steps a)-b), a)-c) or steps a) - d) of the method of claim 1.
